# EUROPEAN PATENT APPLICATION

(11) **EP 4 424 793 A1**
(43) Date of publication of application: **04.09.2024**
(21) Application number: 21962089.5
(22) Date of filing: 09.11.2021
(51) Int. Cl.: C09K 11/02, A61K 103/00, C09K 11/67, A61K 51/02, A61K 103/32, A61K 103/30, A61K 103/20

(54) **RADIOACTIVE MEDICAL ISOTOPE LABELED RARE-EARTH DOPED NANOMATERIAL, PET IMAGING DIAGNOSIS AND TREATMENT AGENT, PREPARATION METHOD THEREFOR AND APPLICATION THEREOF**

(30) Priority: 25.10.2021 CN 202111243137
(71) Applicant: Amonmed Rare Earth Biotechnology (Hebei) Co., LTD, Hebei (CN); Hong, Maochun, Shangjie, Minhou, Fuzhou (CN); Liu, Yongsheng, Shangjie, Minhou, Fuzhou (CN); Mindu Innovation Lab, Fuzhou, Fujian 350108 (CN)
(72) Inventor: HONG, Maochun, Fuzhou, Fujian 350002 (CN); LI, Guowei, Fuzhou, Fujian 350002 (CN); JIANG, Shihui, Fuzhou, Fujian 350002 (CN); LIU, Yongsheng, Fuzhou, Fujian 350002 (CN)
(74) Representative: Gille Hrabal Partnerschaftsgesellschaft mbB Patentanwälte
(86) International application number: PCT/CN2021/129577
(87) International publication number: WO 2023/070743

(57) **Abstract**

A radioactive medical isotope labeled rare-earth doped nanomaterial, a PET imaging diagnosis and treatment agent, a preparation method therefor and an application thereof. The radioactive medical isotope labeled rare-earth doped nanomaterial comprises an inner core. The structural general formula of the inner core is MₓT_{y}F_{x+4y}:Ln/R, wherein M is an alkali metal element, and T is a transition metal element and selected from one or more of Ti, Zr and/or Hf; 1 ≤ x ≤ 7, 1 ≤ y ≤ 6; Ln is a rare earth element and stable isotope; R is a radioactive medical isotope having radioactivity and selected from one or more of ⁹⁰Y, ¹⁷⁷Lu, ¹⁵³Sm, ²²³Ra, ¹¹¹In and, ⁸⁹Zr. The radioactive medical isotope labeled rare-earth ion doped nanomaterial can realize deep tissue penetration high-resolution rare earth fluorescence and global PET multimodal medical images, and is a multifunctional inorganic nano rare earth fluorescent PET imaging agent capable of being effectively degraded and removed in an organism.

## Description

The present application claims priority to a prior application with the patent application No. 202111243137.6 and entitled "RADIOACTIVE MEDICAL ISOTOPE LABELED RARE-EARTH DOPED NANOMATERIAL AND PET IMAGING DIAGNOSIS AND TREATMENT AGENT, PREPARATION METHOD THEREFOR AND APPLICATION THEREOF" filled with China National Intellectual Property Administration on October 25, 2021, which is incorporated herein by reference in its entirety.

### TECHNICAL FIELD

The present disclosure belongs to the technical field of nano-imaging agents, and relates to a radioactive medical isotope labeled rare-earth doped nanomaterial and a Positron Emission Tomography (PET) imaging diagnosis and treatment agent, and particularly to a biodegradable-in-vivo radioactive medical isotope labeled rare-earth doped inorganic nanomaterial and a PET imaging diagnosis and treatment agent, a preparation method therefor and use thereof.

### BACKGROUND

According to the research in recent years, cancer, neurodegenerative diseases, cardiovascular and cerebrovascular diseases and the like have become major factors that seriously threaten people's health. PET diagnosis and treatment taking unique advantages of medical isotopes is an indispensable and important means of improving health. Most of the current PET imaging agents label targeted biomolecules (such as proteins, polypeptides or monoclonal antibodies) by medical isotopes. Although such imaging agents have good targeting properties, they usually have only a single imaging or treatment effect and cannot meet the diagnosis requirements of some complex diseases. The integration of diagnosis and treatment by using an inorganic nanomaterial as a pharmaceutical carrier to load various drug probe molecules is a feasible method for solving the problem.

The rare-earth doped inorganic nanomaterial, as a new generation of fluorescent probe for biological imaging, holds significant promise in developing non-invasive imaging agents due to the characteristics of low biotoxicity, no background fluorescence, photobleaching resistance, deep optical penetration depth and the like. More importantly, the rare-earth doped inorganic nanomaterial is easy to be doped with medical isotopes (such as ⁸⁹Zr, ⁹⁰Y, and ¹⁷⁷Lu) and easy to modify targeting ligands on the surface, and combined with the luminescence characteristics of the material, it can easily realize the multi-modal imaging effect and targeted elimination of the focus.

However, all of the existing rare-earth doped inorganic nanomaterials, e.g., the most representative β-NaYF₄, are non-biodegradable *in vivo* and aggregate in organisms in large quantities, and they cannot be effectively removed from the organisms in a harmless manner, which makes their transformation for clinical use difficult.

Therefore, a biodegradable-in-vivo rare-earth doped inorganic nano-sized pharmaceutical carrier is designed to be used for loading medical isotopes to realize accurate diagnosis and treatment of the focus under deep-tissue-penetrating high-resolution rare-earth fluorescence and global PET multimodal medical image navigation, which will be of great practical significance and clinical value in the diagnosis and treatment application of diseases.

### CONTENTS OF THE DISCLOSURE

In order to solve the technical problems described above, the present disclosure provides a nanomaterial comprising an inner core.

The inner core has a structural general formula of MₓT_{y}F_{x+4y}:Ln/R; wherein,
M is an alkali metal element selected from one or more of Li, Na, K, Rb, and Cs;
T is a transition metal element selected from one or more of Ti, Zr, and Hf;
1 ≤ x ≤ 7, e.g., 1 ≤ x ≤ 4 or 5 ≤ x ≤ 7, exemplarily x = 1, 2, 3, 4, 5, 6, or 7;
1 ≤ y ≤ 6, e.g., 1 ≤ y ≤ 2 or4 ≤ y ≤ 6, exemplarily y = 1, 2, 3, 4, 5, or 6;
Ln is a stable isotope of rare-earth elements selected from one or more of Y, Sc, Yb, Er, Tm, Ho, Gd, Eu, Tb, Sm, Dy, Ce, Nd, La, Pr, and Lu, preferably one or more of Yb, Er, Tm, Ho, Eu, Nd, Y, and Lu, e.g., Yb/Er co-doped;
R is a radioactive medical isotope selected from one or more of ⁹⁰Y, ¹⁷⁷Lu, ¹⁵³Sm, ²²³Ra, ¹¹¹In, and ⁸⁹Zr, preferably one or more of ⁹⁰Y, ¹⁷⁷Lu, ¹¹¹In, and ⁸⁹Zr, exemplarily ⁸⁹Zr.

According to an embodiment of the present disclosure, R is distributed at least inside a crystal of the inner core. For example, R is distributed in the inner core at an abundance of at least 5 mCi/g. For another example, R is distributed in the inner core at an abundance of at least 6 mCi/g.

According to an embodiment of the present disclosure, in MₓT_{y}F_{x+4y}:Ln/R, each element is present in an ionic state.

According to an embodiment of the present disclosure, the inner core can be selected from materials having the following structural formula: K₃ZrF₇:Yb/Er/⁸⁹Zr, K₂ZrF₆:Yb/Er/⁸⁹Zr, KZrF₅:Yb/Er/⁸⁹Zr, K₃HfF₇:Yb/Er/⁸⁹Zr, K₂HfF₆:Yb/Er/⁸⁹Zr, K₃Zr_{0.5}Hf_{0.5}F₇:Yb/Er/⁸⁹Zr, Na₃ZrF₇:Yb/Er/⁸⁹Zr, Na₂ZrF₆:Yb/Er/⁸⁹Zr, Na₅Zr₂F₁₃:Yb/Er/⁸⁹Zr, Na₇Zr₆F₃₁:Yb/Er/⁸⁹Zr, Na₃HfF₇:Yb/Er/⁸⁹Zr, Na₅Hf₂F₁₃:Yb/Er/⁸⁹Zr, Na₃Zr_{0.5}Hf_{0.5}F₇:Yb/Er/⁸⁹Zr, Na₇Hf₆F₃₁:Yb/Er/⁸⁹Zr, Li₄ZrF₈:Yb/Er/⁸⁹Zr, Li₂ZrF₆:Yb/Er/⁸⁹Zr, Li₃Zr₄F₁₉:Yb/Er/⁸⁹Zr, Li₂HfF₆:Yb/Er/⁸⁹Zr, Rb₅Zr₄F₂₁:Yb/Er/⁸⁹Zr, Rb₂ZrF₆:Yb/Er/⁸⁹Zr, Cs₂ZrF₆:Yb/Er/⁸⁹Zr, RbHfF₅:Yb/Er/⁸⁹Zr, Rb₂HfF₆:Yb/Er/⁸⁹Zr, Cs₂HfF₆:Yb/Er/⁸⁹Zr, K₂TiF₆:Yb/Er/⁸⁹Zr, or Na₂TiF₆:Yb/Er/⁸⁹Zr; exemplarily K₃ZrF₇:Yb/Er/⁸⁹Zr, Na₇Zr₆F₃₁:Yb/Er/⁸⁹Zr, or Na₅Hf₂F₁₃:Yb/Er/⁸⁹Zr.

According to an embodiment of the present disclosure, the inner core has a crystalline structure. For example, the inner core has a trigonal, cubic, tetragonal, or monoclinic phase structure.

According to an embodiment of the present disclosure, the inner core has a dimension of 5-100 nm, e.g., 20-90 nm, exemplarily 23.4 nm, 27.5 nm, or 83.3 nm.

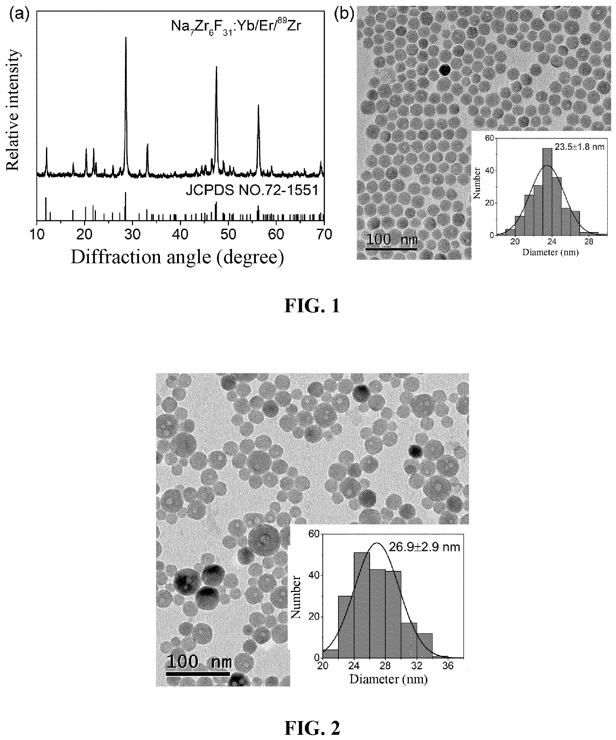

According to an embodiment of the present disclosure, the inner core has an X-ray powder diffraction pattern substantially as shown in FIG. 1(a), FIG. 3(a), or FIG. 4(a).

According to an embodiment of the present disclosure, the inner core has a transmission electron micrograph substantially as shown in FIG. 1(b), FIG. 3(b), or FIG. 4(b).

According to an embodiment of the present disclosure, the nanomaterial further comprises a functional shell layer. The functional shell layer is located on the surface of the inner core, for example, the surface of the inner core is partially or completely coated with the functional shell layer.

According to an embodiment of the present disclosure, the functional shell layer can be a functional shell layer composed of an organic material (e.g., an organic ligand), a functional shell layer composed of an inorganic material, or an organic/inorganic hybrid functional shell layer, exemplarily a functional shell layer composed of an organic ligand. It can be understood by those skilled in the art that the functional shell layer can be selected according to the usage requirements of the scenario.

According to an embodiment of the present disclosure, the organic ligand can be selected from a water-soluble functional ligand, an oil-soluble functional ligand (such as oleic acid), an amphiphilic functional ligand (such as liposome) or other functional ligands with pharmaceutical functions (such as a biological small molecule, a biological large molecule, and an antibody), exemplarily a water-soluble functional ligand. For example, the water-soluble functional ligand is selected from one or more of methoxypolyethylene glycol alendronate (MPEG-ALE), polyacrylic acid (PAA), and distearoyl phosphatidyl ethanolamine-polyethylene glycol (DSPE-PEG).

According to an embodiment of the present disclosure, the functional shell layer has a thickness of 1-10 nm, exemplarily 2 nm, 3.5 nm, 5 nm, or 8 nm.

According to an embodiment of the present disclosure, the nanomaterial has a dimension greater than that of the inner core. For example, the nanomaterial has a dimension in a range of 7-100 nm, preferably 25-55 nm, e.g., has a dimension of 10 nm, 20 nm, 27 nm, 30 nm, 40 nm, 50 nm, 60 nm, or 80 nm.

According to an embodiment of the present disclosure, the nanomaterial has the characteristic of decomposition when exposed to water. The speed of decomposition is affected by the type of the inner core, the concentration of an aqueous solution, and acidity or basicity (pH value), and different types of inner cores can be selected and different concentrations of aqueous solutions can be prepared to meet the usage requirements of different environments. For example, the time for complete decomposition of a nanomaterial with an inner core of K₃ZrF₇:Yb/Er/⁸⁹Zr immersed in deionized water is less than 1 h; the time for complete decomposition of a nanomaterial with an inner core of Na₇Zr₆F₃₁:Yb/Er/⁸⁹Zr immersed in deionized water is more than 3 days. For another example, the time for complete decomposition of a nanomaterial with an inner core of K₃ZrF₇:Yb/Er/⁸⁹Zr in a biological subcutaneous tissue with neutral pH is less than 1 h; the time for complete decomposition in a weakly acidic tumor tissue is 2-4 h.

According to an embodiment of the present disclosure, the nanomaterial has relatively low biotoxicity.

According to an embodiment of the present disclosure, the nanomaterial has a transmission electron micrograph substantially as shown in FIG. 2.

According to an embodiment of the present disclosure, the preparation method for the nanomaterial can be selected from a coprecipitation method, a thermal decomposition method, a hydrothermal method, a sol-gel method, or the like, exemplarily a coprecipitation method.

According to an embodiment of the present disclosure, the coprecipitation method for preparing the nanomaterial comprises the following steps:
(1) using a stable isotope T source, a non-radioactive rare-earth source, a radioactive isotope source, long-alkyl-chain organic acid, octadecene or trioctylamine, an alkali metal source, and a fluorine source as starting materials, and obtaining an inner core by a coprecipitation method; and
(2) optionally, modifying the inner core with a functional shell layer to obtain the nanomaterial.

According to an embodiment of the present disclosure, both the inner core and the functional layer have the meaning as indicated above.

According to an embodiment of the present disclosure, a T element in the stable isotope T source is one or more of Ti, Zr, and Hf, and is exemplarily selected from Zr or/and Hf.

According to an embodiment of the present disclosure, the stable isotope T source is selected from one or more of the following: zirconium acetylacetonate, hafnium acetylacetonate, zirconium acetate, titanium tetrachloride, zirconium chloride, hafnium chloride, and the like, preferably one or more of zirconium acetate, zirconium acetylacetonate, hafnium acetylacetonate, zirconium chloride, and hafnium chloride.

According to an embodiment of the present disclosure, the non-radioactive rare-earth source provides the inner core with a rare-earth ion stable isotope Ln.

According to an embodiment of the present disclosure, the non-radioactive rare-earth source is selected from one or more of the following: acetate, chloride, and nitrate of the rare-earth ion stable isotope Ln, preferably acetate and/or chloride of the rare-earth ion stable isotope Ln, further preferably acetate and/or chloride of Yb, Er, Tm, Ho, Eu, Nd, Y, and Lu, e.g., acetate of Yb, chloride of Yb, acetate of Er, and/or chloride of Er.

Preferably, when the non-radioactive rare-earth source is selected from a Yb ion stable isotope source and an Er ion stable isotope source, the Yb ion and the Er ion are in a molar ratio of (10-30):(0.5-4), e.g., (15-20):(1-3).

According to an embodiment of the present disclosure, the radioactive isotope source provides the inner core with an R element.

According to an embodiment of the present disclosure, the radioactive isotope source is selected from an oxalate salt of a radioactive isotope and/or chloride of a radioactive isotope, exemplarily chloride of a radioactive isotope. As an example, the radioactive isotope source is ⁸⁹ZrCl.

According to an embodiment of the present disclosure, the long-alkyl-chain organic acid is selected from one or more of the following: caprylic acid, lauric acid, oleic acid, and the like, exemplarily oleic acid.

According to an embodiment of the present disclosure, the alkali metal source provides the inner core with an element M. For example, the alkali metal source is selected from one or more of hydroxide, oleate, acetate, fluoride, and hydrogenfluoride containing the M element, preferably hydroxide, fluoride, or acetate containing the M element, exemplarily hydroxide, fluoride, or acetate of K and/or Na.

According to an embodiment of the present disclosure, the fluorine source is selected from one or more of ammonium fluoride, sodium fluoride, potassium fluoride, sodium hydrogenfluoride, potassium hydroxide, and trifluoroacetate, preferably ammonium fluoride, sodium fluoride, potassium fluoride, sodium hydrogenfluoride, or potassium hydroxide.

According to an embodiment of the present disclosure, the inner core is oil-soluble.

According to an embodiment of the present disclosure, the stable isotope T source, the non-radioactive rare-earth source, the long-alkyl-chain organic acid, and the octadecene are in a mole-to-volume ratio of (0.1-0.49) mmol : (0.01-0.4) mmol : (5-10) mL : (5-30) mL; preferably, the stable isotope T source, the non-radioactive rare-earth source, the long-alkyl-chain organic acid, and the octadecene are in a mole-to-volume of (0.2-0.3) mmol : (0.01-0.1) mmol : (6-12) mL : (8-20) mL.

According to an embodiment of the present disclosure, the stable isotope T source, the non-radioactive rare-earth source, the long-alkyl-chain organic acid, and the trioctylamine are in a mole-to-volume ratio of (0.1-0.49) mmol : (0.01-0.4) mmol : (5-10) mL : (5-30) mL; preferably, the stable isotope T source, the non-radioactive rare-earth source, the long-alkyl-chain organic acid, and the trioctylamine are in a mole-to-volume of (0.2-0.3) mmol : (0.01-0.1) mmol : (6-12) mL : (8-20) mL.

According to an embodiment of the present disclosure, the radioactive isotope source and the stable isotope T source are in a radioactivity-to-mole ratio of (1-10) mCi : (0.1-0.49) mmol; preferably, the radioactive isotope source and the stable isotope T source are in a radioactivity-to-mole ratio of (2-8) mCi : (0.2-0.4) mmol.

According to an embodiment of the present disclosure, the step (1) comprises:
(a) mixing the stable isotope T source, the non-radioactive rare-earth source, the radioactive isotope source, the long-alkyl-chain organic acid, and the octadecene, or mixing the stable isotope T source, the non-radioactive rare-earth source, the radioactive isotope source, the long-alkyl-chain organic acid, and the trioctylamine in a reaction container until dissolution to obtain a solution a; and
(b) adding the alkali metal source and the fluorine source to the solution a, and performing a coprecipitation reaction to obtain the inner core.

According to an embodiment of the present disclosure, the mixing in the step (a) is performed under conditions of an inert gas atmosphere and stirring at a temperature of 140-165 °C, preferably at a temperature of 150-160 °C.

Preferably, the mixing in the step (a) is performed for 0.3-1 h, e.g., 0.5-1 h.

Preferably, after mixing until dissolution in the step (a), the step (1) further comprises a step of cooling the solution to room temperature.

Preferably, the solution a is a bright yellow solution.

According to an embodiment of the present disclosure, before adding the alkali metal source and the fluorine source to the solution a in the step (b), the step (1) further comprises a step of dissolving the alkali metal source and the fluorine source in a solvent. Preferably, the solvent is ethanol or methanol, e.g., methanol.

According to an embodiment of the present disclosure, in the step (b), the alkali metal source, the fluorine source, and the solvent are in a proportion of (0.05-5) mmol:(0.2-7) mmol:(1-12) mL, and preferably, the alkali metal source, the fluorine source, and the solvent are in a proportion of (0.3-3) mmol:(2-4) mmol:(3-10) mL.

According to an embodiment of the present disclosure, after adding the alkali metal source and the fluorine source to the solution a for a reaction in the step (b), the step (1) further comprises a step of removing the solvent. Preferably, the solvent is removed by evaporation under an inert gas atmosphere. For example, methanol is removed by heating to 60-100 °C under an inert gas atmosphere and keeping the temperature for 30 min.

Preferably, in the step (b), the mixture is heated to the reaction temperature after the solvent is removed.

According to an embodiment of the present disclosure, the coprecipitation reaction in the step (b) is performed at a temperature of 260-340 °C for 0.3-2 h. Preferably, the reaction is performed at a temperature of 280-320 °C for 0.5-1.5 h. Further preferably, the reaction is performed at a temperature of 300-320 °C for 1-1.2 h.

Preferably, the coprecipitation reaction in the step (b) is performed under an inert gas atmosphere with vigorous stirring.

Preferably, after the coprecipitation reaction in the step (b) is completed, the step (1) further comprises steps of natural cooling to room temperature, precipitation, and washing. Preferably, after washing, the step (1) further comprises steps of centrifugal separation and purification.

According to an embodiment of the present disclosure, the step (2) can be modifying the inner core with different functional shell layers according to the usage requirements, exemplarily modifying the inner core with a functional shell layer composed of a water-soluble ligand. For example, the step of modifying comprises: dispersing the inner core in a mixed solvent, adding trimethyloxonium tetrafluoroborate for a reaction to remove oil-soluble substances on the surface of the inner core, precipitating the resulting solution, re-dispersing in N,N-dimethylformamide (DMF), adding a functional ligand solution (preferably a water-soluble functional ligand solution) under stirring, and performing a reaction to obtain the nanomaterial. For example, the reaction is performed for 8-12 h.

According to an embodiment of the present disclosure, the functional ligand can be an organic material (e.g., an organic ligand), an inorganic material, or an organic/inorganic hybrid material. Preferably, the organic ligand has the meaning as described above.

According to an embodiment of the present disclosure, the mixed solvent is a mixed solution of cyclohexane and N,N-dimethylformamide (DMF).

Preferably, the reaction after adding the trimethyloxonium tetrafluoroborate is performed for 15-60 min, more preferably 30-40 min.

Preferably, the precipitation comprises: adding excessive toluene to the solution to precipitate the inner core from which the oil-soluble substances have been removed.

According to an embodiment of the present disclosure, the inner core (calculated as the T ion, preferably as a Zr/Hf ion), trimethyloxonium tetrafluoroborate ((CH₃)₃OBF₄), cyclohexane, and N,N-dimethylformamide (DMF) are in a mole-to-volume ratio of (0.05-0.2) mmol : (0.2-0.7) mmol : (2.5-10) mL : (2.5-10) mL, preferably (0.1-0.15) mmol : (0.3-0.5) mmol : (5-6) mL : (5-6) mL.

According to an embodiment of the present disclosure, the inner core (calculated as the T ion, preferably as a Zr/Hf ion), N,N-dimethylformamide (DMF) and functional ligand are in a mole-to-volume ratio of (0.05-0.2) mmol : (6-24) mL : (0.05-0.2) mmol, preferably (0.1-0.2) mmol : (10-20) mL : (0.1-0.2) mmol.

According to an embodiment of the present disclosure, the step (2) further comprises a step of performing precipitation of the reaction solution and washing after the reaction. Preferably, the precipitation is performed by high-speed centrifugation.

According to an embodiment of the present disclosure, the step (2) further comprises a step of re-dispersing the product after washing, e.g., in normal saline. Preferably, the step (2) further comprises a step of performing sterile filtration on the dispersed solution after dispersing.

The present disclosure also provides use of the nanomaterial described above as a rare-earth fluorescent PET imaging agent, preferably as a specific rare-earth fluorescent PET imaging agent and/or therapeutic agent for diseases including, but not limited to cancer, neurodegenerative diseases, and cardiovascular and cerebrovascular diseases.

The present disclosure also provides a PET imaging agent, which comprises the nanomaterial described above.

The present disclosure also provides use of the PET imaging agent described above as a specific rare-earth fluorescent PET imaging agent and/or therapeutic agent for diseases including, but not limited to cancer, neurodegenerative diseases, and cardiovascular and cerebrovascular diseases.

### Beneficial Effects of Present Disclosure

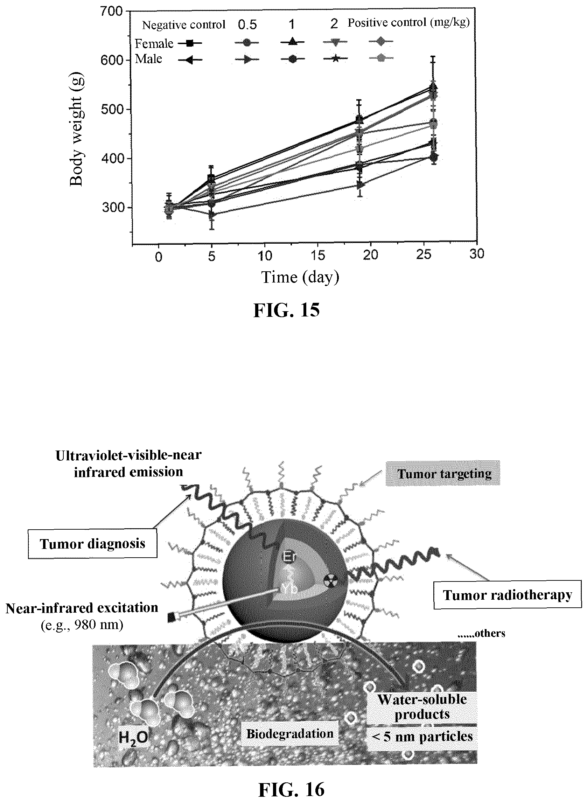

In the present disclosure, by usingng an inorganic transition metal element (titanium/zirconium/hafnium)-base alkali metal fluoride system as a matrix material, utilizing an improved high-temperature coprecipitation method to dope non-radioactive rare-earth ions and radioactive medical isotopes into crystal lattices to obtain a nano-inner core, and performing a functional modification on the inner core, a class of a radioactive medical isotope labeled inorganic multifunctional rare-earth fluorescent nanomaterial that can be effectively degraded and removed *in vivo* is obtained, which can be used as a PET imaging agent (see FIG. 16). The nanomaterial has the following advantages:
1) The synthesis method is easy to control and has good repeatability, the prepared nanomaterial has good dispersibility and uniformity, and the doping of rare-earth ions can realize the luminescence from ultraviolet to near-infrared light regions under the excitation of near-infrared light.
2) The transition metal element (titanium/zirconium/hafnium)-based alkali metal fluoride material has the material characteristics of easily being doped. For example, ⁸⁹Zr is a radioactive isotope of ⁹⁰Zr, which belongs to group IVB with Ti and Hf and is easily doped into the crystal lattice, and ⁸⁹Zr has a proper physical half-life (78.4 h), low positron energy, and stable PET imaging quality, making it an ideal PET imaging nuclide. ⁸⁹Zr is added during preparation of an oil-soluble nano-inner core, so that ⁸⁹Zr is doped into the crystal lattice of the matrix material. Not only is the loading capacity of ⁸⁹Zr greater than that of ⁸⁹Zr in a mode of directly chelating a small/large molecule, but also ⁸⁹Zr can stably exist in the crystal material.
3) The surface of the nanomaterial can be selectively covered with various functional shell layers, which can be divided into a functional shell layer composed of an organic material, a functional shell layer composed of an inorganic material, or an organic/inorganic hybrid functional shell layer, thereby meeting different usage requirements in actual situations.
4) The nanomaterial of the present disclosure can be decomposed into a water-soluble fluozirconate/fluohafnate ion ([T_{y}F_{x+4y}]^{x-}, wherein T is Zr or Hf), and provides a unique acidity or basicity-dependent water degradation capability. It can also provide up-conversion luminescence and PET radiography while being decomposed in an aqueous environment. Due to the special hydrolysis characteristic of the matrix material, the PET imaging agent can be hydrolyzed and removed in organisms quickly, and provides the possibility of use in organisms.
5) The decomposition of the nanomaterial prepared according to the present disclosure as a PET imaging agent will be inhibited to a certain extent in a tumor environment of weak acidity (pH 5-6). Due to the prolonged hydrolysis of the material in an environment of weak acidity, it can exist in a tumor environment of weak acidity for a longer period than in other *in vivo* environments, and can be used in specific treatment and labeling of tumors of weak acidity.
6) Due to the nano-inner core with relatively low cytotoxicity and biotoxicity, the PET imaging agent prepared according to the present disclosure exhibits good biosafety, can be safely used in organisms, is not prone to massive aggregation in organisms, and is degradable, so that it can be used as a brand-new inorganic nano-fluorescent PET imaging material with potential application prospects in the fields of drug transportation, PET immunoimaging, bioimaging, and the like. The accurate diagnosis and treatment of the focus under deep-tissue-penetrating high-resolution rare-earth fluorescence and global PET multimodal medical image navigation achieved by loading radioactive medical isotopes will be of great practical significance and clinical value in the diagnosis and treatment application of diseases.

### BRIEF DESCRIPTION OF THE DRAWINGS

FIG. 1 shows an X-ray powder diffraction pattern of a trigonal phase Na₇Zr₆F₃₁:Yb/Er/⁸⁹Zr-based nano-inner core, and corresponding transmission electron micrograph and particle size distribution histogram of Example 1 according to the present disclosure.
FIG. 2 shows a transmission electron micrograph and a particle size distribution histogram of a trigonal phase Na₇Zr₆F₃₁:Yb/Er/⁸⁹Zr-based nanomaterial modified with a ligand of Example 1 according to the present disclosure.
FIG. 3 shows an X-ray powder diffraction pattern of a cubic phase K₃ZrF₇:Yb/Er/⁸⁹Zr-based nanomaterial, and corresponding transmission electron micrograph and particle size distribution histogram of Example 2 of the present disclosure.
FIG. 4 shows an X-ray powder diffraction pattern of a monoclinic phase Na₅Hf₂F₁₃:Yb/Er/⁸⁹Zr-based nano-inner core, and corresponding transmission electron micrograph and schematic morphology of Example 3 according to the present disclosure.
FIG. 5 shows an up-conversion emission spectrum (excitation wavelength of 980 nm) of the Na₇Zr₆F₃₁:Yb/Er/⁸⁹Zr-based nanomaterial of Example 1 dispersed in deionized water, and a corresponding luminescence photograph in Experimental Example 1 according to the present disclosure.
FIG. 6 shows a curve of change of the up-conversion luminous intensity of the Na₇Zr₆F₃₁:Yb/Er/⁸⁹Zr-based nanomaterial of Example 1 dispersed in deionized water over time at a concentration of 2 mg/mL under the excitation of 980 nm laser, and corresponding luminescence photographs in Experimental example 1 according to the present disclosure.
FIG. 7 shows an up-conversion emission spectrum (excitation wavelength of 980 nm) of the K₃ZrF₇:Yb/Er/⁸⁹Zr-based nanomaterial of Example 2 dried into a powder, and a corresponding luminescence photograph in Experimental Example 1 according to the present disclosure.
FIG. 8 shows a curve of change of the up-conversion luminous intensity of the K₃ZrF₇:Yb/Er/⁸⁹Zr-based nanomaterial powder of Example 2 added to deionized water over time under the excitation of 980 nm laser, and corresponding luminescence photographs, as well as an X-ray powder diffraction pattern of the nanomaterial decomposed in deionized water in Experimental Example 1 according to the present disclosure.
FIG. 9 is a schematic diagram showing the subcutaneous imaging effect of the K₃ZrF₇:Yb/Er/⁸⁹Zr-based nano-imaging agent of Example 2 in nude mice in Experimental Example 2 according to the present disclosure.
FIG. 10 is a schematic diagram showing the PET imaging effect of the Na₇Zr₆F₃₁:Yb/Er/⁸⁹Zr-based nano-imaging agent of Example 1 in ICR mice 1 h, 3 h, 6 h, 72 h, and 120 h after administration through the tail vein (at a concentration of 150 mg/kg and a radioactive dose of 4.26 mCi/kg) in Experimental Example 3 according to the present disclosure.
FIG. 11 is a schematic diagram showing the PET imaging effect of the Na₇Zr₆F₃₁:Yb/Er/⁸⁹Zr-based nano-imaging agent of Example 1 in ICR mice 1 h, 3 h, and 6 h after administration through the tail vein (at a concentration of 75 mg/kg and a radioactive dose of 2.13 mCi/kg) in Experimental Example 3 according to the present disclosure.
FIG. 12 is a graph showing the cytotoxicity results of the Na₇Zr₆F₃₁:Yb/Er/⁸⁹Zr-based nano-imaging agent of Example 1 in Experimental Example 4 according to the present disclosure.
FIG. 13 is a graph showing tissue sections of the major organs of ICR mice after an ICR mouse acute toxicity test of the Na₇Zr₆F₃₁:Yb/Er/⁸⁹Zr-based nano-imaging agent of Example 1 in Experimental Example 4 according to the present disclosure.
FIG. 14 is a graph showing tissue sections of the major organs of SD rats after long-term toxicity test of the Na₇Zr₆F₃₁:Yb/Er/⁸⁹Zr-based nano-imaging agent of Example 1 in Experimental Example 4 according to the present disclosure.
FIG. 15 is a graph showing the change of body weight of guinea pigs in the guinea pig allergy test of the Na₇Zr₆F₃₁:Yb/Er/⁸⁹Zr-based nano-imaging agent of Example 1 in Experimental Example 4 according to the present disclosure.
FIG. 16 is a schematic diagram showing the principle of diagnosis, treatment, and degradation of the Na₇Zr₆F₃₁:Yb/Er/⁸⁹Zr-based nano-imaging agent of Example 1 in Experimental Example 4 according to the present disclosure.

### MODES FOR CARRYING OUT THE DISCLOSURE

The technical solutions of the present disclosure will be further described in detail with reference to the following specific examples. It should be understood that the following examples are merely exemplary illustration and explanation of the present disclosure, and should not be construed as limiting the protection scope of the present disclosure. All techniques implemented based on the afore-mentioned contents of the present disclosure are encompassed within the protection scope of the present disclosure.

Unless otherwise stated, the starting materials and reagents used in the following examples are all commercially available products, or can be prepared by using known methods. The instruments involved in the following examples are as follows:
An X-ray powder diffractometer (instrument model: MiniFlex2, manufacturer: Rigaku), a transmission electron microscope (instrument model: TECNAI G2 F20, manufacturer: FEI), a spectrometer (instrument model: FLS980, manufacturer: Edinburgh), a camera (instrument model: EOS 5D, manufacturer: Canon), an intelligent calibrator (instrument model: China Nuclear FH463B, manufacturer: Guangzhou Yitongxing Instrument Co., Ltd.), a CCD imaging system (manufacturer: ANDOR), a positron emission/X-ray transmission computed tomographic scanner for living small animals (instrument model: IRIS PET/CT, manufacturer: Inviscan).

### Comparative Example 1: Preparation of β-NaYF₄:20%Yb/2%Er-Based Nanomaterial

The β-NaYF₄:20%Yb/2%Er-based nanomaterial was prepared based on a coprecipitation method. Yttrium acetate, ytterbium acetate, and erbium acetate according to a stoichiometric ratio of 0.39 mmol : 0.1 mmol : 0.01 mmol were added to a three-necked flask at room temperature, and then 5 mL of oleic acid and 16 mL of octadecene were added as solvents to completely dissolve the solids after being heated to 150-160 °C under an inert gas atmosphere, followed by natural cooling to room temperature to obtain a clear solution. A 10 mL of methanolic solution of 1.25 mmol sodium hydroxide and 2 mmol ammonium fluoride was added to the above solution. The mixture was heated to 65-80 °C under an inert gas atmosphere and kept at this temperature until methanol was completely removed, heated to 100-110 °C and kept at this temperature until water was completely removed, and then heated to 300 °C under an inert gas atmosphere and kept at this temperature for 40-60 min, followed by natural cooling to room temperature. The mixture was precipitated and washed to obtain the β-NaYF₄:20%Yb/2%Er-based nanomaterial.

### Example 1: Na₇Zr₆F₃₁:Yb/Er/⁸⁹Zr-Based Rare-Earth Fluorescent PET Nano-Imaging Agent and Preparation Thereof

(1) The Na₇Zr₆F₃₁:Y⁻b/Er/⁸⁹Zr-based nano-inner core was prepared based on a coprecipitation method.

Zirconium acetate, ytterbium acetate, and erbium acetate according to a stoichiometric ratio of 0.39 mmol : 0.1 mmol : 0.01 mmol were added to a three-necked flask at room temperature, 10 mL of oleic acid and 10 mL of octadecene were added as solvents, and then 6 mCi ⁸⁹ZrCl was added. The mixture was heated to 150-165 °C under an inert gas atmosphere and kept at this temperature for 25-50 min, followed by cooling to room temperature to obtain a bright yellow solution a.

A 5 mL of methanolic solution of 0.44 mmol sodium hydroxide and 2.08 mmol ammonium fluoride was added to the above solution a. The mixture was heated to 70-80 °C under an inert gas atmosphere and kept at this temperature for 30 min to remove methanol, then heated to 300-320 °C, and vigorously stirred for a reaction for 0.5-1.5 h, followed by natural cooling to room temperature. The mixture was precipitated and washed to obtain a trigonal phase Na₇Zr₆F₃₁:Yb/Er/⁸⁹Zr-based nano-inner core. The X-ray powder diffraction pattern of the nano-inner core is shown in FIG. 1(a), 89Zr is doped into the crystal lattice, and the nano-inner core has a dimension of about 23.5 nm (shown in FIG. 1(b)).

(2) The Na₇Zr₆F₃₁:Yb/Er/⁸⁹Zr-basednano-inner core was subjected to surface ligand modification.

The Na₇Zr₆F₃₁:Yb/Er/⁸⁹Zr-basednano-inner core (containing about 0.1 mmol Zr ions) was dispersed in a mixed solution of 5 mL of cyclohexane and 5 mL of N,N-dimethylformamide (DMF) at room temperature, and 40-60 mg of trimethyloxonium tetrafluoroborate ((CH₃)₃OBF₄) was rapidly added to the mixed solution under stirring for a reaction for 20-45 min to remove oil-soluble substances on the surface of the nano-inner core. Excessive toluene was added to the resulting solution for precipitation. The precipitant was redispersed in N,N-dimethylformamide (DMF), and 0.125 mmol methoxypolyethylene glycol alendronate (MPEG-ALE) was added under stirring for a reaction for 8-12 h. The reaction solution was precipitated by high-speed centrifugation and washed to obtain a precipitation product, namely a hydrophilic Na₇Zr₆F₃₁:Yb/Er/⁸⁹Zr-based nanomaterial. As shown in FIG. 2, the resulting hydrophilic Na₇Zr₆F₃₁:Yb/Er/⁸⁹Zr-based nanomaterial has good dispersibility, and has a dimension of about 26.9 nm, which is about 3.3 nm greater than that of the nano-inner core before modification, which is supposed to be caused by the thickness of the surface ligand.

The precipitation product was dispersed in normal saline, and the dispersion was subjected to sterile filtration. The hydrophilic Na₇Zr₆F₃₁:Yb/Er/⁸⁹Zr-based rare-earth fluorescent nanomaterial was used as a Na₇Zr₆F₃₁:Yb/Er/⁸⁹Zr-based rare-earth fluorescent PET nano-imaging agent.

### Example 2: K₃ZrF₇:Yb/Er/⁸⁹Zr-Based Rare-Earth Fluorescent PET Nano-Imaging Agent and Preparation Thereof

The K₃ZrF₇:Yb/Er/⁸⁹Zr-based nano-inner core was prepared based on a coprecipitation method. Zirconium acetate, ytterbium acetate, and erbium acetate according to a stoichiometric ratio of 0.39 mmol : 0.1 mmol : 0.01 mmol were added to a three-necked flask at room temperature, then 8.5 mL of oleic acid and 17 mL of octadecene were added as solvents, and 6 mCi ⁸⁹ZrCl was added under an inert gas atmosphere. The mixture was heated to 150-160 °C and kept at this temperature for 25-50 min, followed by cooling to room temperature to obtain a bright yellow solution a.

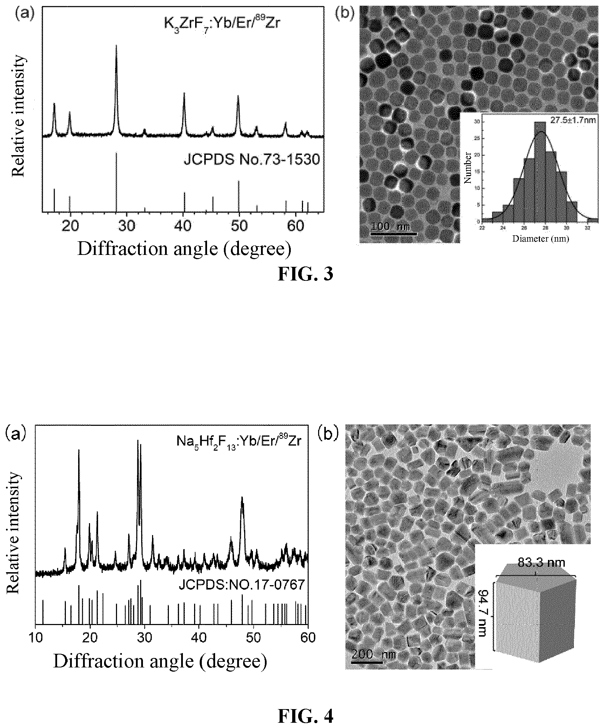

A 10 mL of methanolic solution with 3 mmol potassium hydroxide and 3.5 mmol ammonium fluoride dissolved therein was added to the bright yellow solution a. The mixture was heated to 60-100 °C under an inert gas atmosphere and kept at this temperature for 30 min to remove methanol, then heated to 300-320 °C, and vigorously stirred for a reaction for 0.5-1 h, followed by natural cooling to room temperature. The mixture was precipitated and washed to obtain a cubic phase K₃ZrF₇:Yb/Er/89Zr-based nano-inner core. The X-ray powder diffraction pattern of the nano-inner core is shown in FIG. 3(a), 89Zr is doped into the crystal lattice, and the nano-inner core has a dimension of about 27.5 nm (shown in FIG. 3(b)). The K₃ZrF₇:Yb/Er/⁸⁹Zr-based inner core is extremely easy to decompose in water, which causes difficulty in water-soluble modification, but an oleic acid molecule covering the surface of the inner core enables the inner core to be dispersed in an oil-soluble solution. Based on this, the K₃ZrF₇:Yb/Er/⁸⁹Zr-based inner core was dispersed in edible peanut oil in this Example to obtain an oil-soluble K₃ZrF₇:Yb/Er/⁸⁹Zr-based rare-earth fluorescent nanomaterial, which was used as a K₃ZrF₇:Yb/Er/⁸⁹Zr-based rare-earth fluorescent PET nano-imaging agent.

### Example 3: Na₅Hf₂F₁₃:Yb/Er/⁸⁹Zr-Based Rare-Earth Fluorescent PET Nano-Imaging Agent and Preparation Thereof

(1) The Na₅Hf₂F₁₃:Yb/Er/⁸⁹Zr-based nano-inner core was prepared based on a coprecipitation method.

Hafnium acetylacetonate, ytterbium acetate, and erbium acetate according to a stoichiometric ratio of 0.19 mmol : 0.008 mmol : 0.002 mmol were added to a three-necked flask at room temperature, then 4 mL of oleic acid and 4 mL of octadecene were added as solvents, and 3 mCi ⁸⁹ZrCl was added under an inert gas atmosphere. The mixture was heated to 150-160 °C and kept at this temperature for 25-50 min, followed by cooling to room temperature to obtain a bright yellow solution a.

A 10 mL of methanolic solution with 0.5 mmol sodium hydroxide and 1.2 mmol ammonium fluoride dissolved therein was added to the bright yellow solution a. The mixture was heated to 70-80 °C under an inert gas atmosphere and kept at this temperature for 30 min to remove methanol, then heated to 300-320 °C, and vigorously stirred for a reaction for 0.5-1 h, followed by natural cooling to room temperature. The mixture was precipitated and washed to obtain a monoclinic phase Na₅Hf₂F₁₃:Yb/Er/⁸⁹Zr-based nano-inner core. The X-ray powder diffraction pattern of the nano-inner core is shown in FIG. 4(a), and 89Zr is doped into the crystal lattice; the nano-inner core has the morphology shown in FIG. 4(b) as a pentagonal prism with a length of 94.7 nm and a diameter of 83.3 nm.

(2) The Na₅Hf₂F₁₃:Yb/Er/⁸⁹Zr-based nano-inner core was subjected to surface ligand modification.

The lipophilic Na₅Hf₂F₁₃:Yb/Er/⁸⁹Zr-based nano-inner core (containing about 0.1 mmol hafnium ions) was dispersed in a mixed solution of cyclohexane and N,N-dimethylformamide (DMF) (volume ratio: 1:1) at room temperature, and 40-60 mg of trimethyloxonium tetrafluoroborate ((CH₃)₃OBF₄) was rapidly added to the mixed solution under stirring for a reaction for 20-45 min to remove oil-soluble substances on the surface of the nano-inner core. Excessive toluene was added to the resulting solution for precipitation. The precipitant was redispersed in N,N-dimethylformamide (DMF), and 0.125 mmol methoxypolyethylene glycol alendronate (MPEG-ALE) was added under stirring for a reaction for 8-12 h. The reaction solution was precipitated by high-speed centrifugation and washed to obtain a hydrophilic Na₅Hf₂F₁₃: Yb/Er/⁸⁹Zr-based nanomaterial.

The hydrophilic Na₅Hf₂F₁₃:Yb/Er/⁸⁹Zr-based nanomaterial was dispersed in normal saline, and the dispersion was subjected to sterile filtration to be used as a Na₅Hf₂F_{I3}: Yb/Er/⁸⁹Zr-based rare-earth fluorescent PET nano-imaging agent.

### Example 4: Verification of Radioactivity of Na₇Zr₆F₃₁:Yb/Er/⁸⁹Zr-Based Nanomaterial

The ⁸⁹Zr labeling rate of the Na₇Zr₆F₃₁:Y⁻b/Er/⁸⁹Zr-based nanomaterial prepared in Example 1 was measured by an intelligent calibrator.

### (1) Theoretical estimation of radioactivity of the prepared Na₇Zr₆F₃₁:Y⁻b/Er/⁸⁹Zr-based nano-imaging agent:

According to the synthesis method of Example 1, 0.083 mmol Na₇Zr₆F₃₁:Yb/Er-based inorganic nano-inner core (wherein Zr + Yb + Er = 0.5 mmol) was doped with 500 µCi ⁸⁹Zr, and 108.1 mg of product would be obtained if the reaction was complete. Considering that the yield of Na₇Zr₆F₃₁:Y⁻b/Er/⁸⁹Zr-based nano-inner core was 40%-50%, it was estimated that the actual yield was 43.24-54.05 mg, and the radioactivity of the product was 200-250 µCi.

The Na₇Zr₆F₃₁:Yb/Er/⁸⁹Zr-based nano-inner core was subjected to further surface water-solubility modification, and 40%-50% of sample would be remained after the surface modification. It was estimated that 17.3-27 mg of the sample would be remained after the surface modification, and 80-125 µCi of radioactivity of the product would be remained.

### (2) Experimental results:

Using 0.083 mmol Na₇Zr₆F₃₁:Y⁻b/Er/⁸⁹Zr-based inorganic nano-inner core doped with 500 µCi ⁸⁹Zr as the starting material for synthesis, 54.05 mg of the product was actually obtained, with a sample yield of 50% and product radioactivity of 218 µCi, so that the labeling rate of the sample should be 218/(500 × 0.5) = 87.2%.

Further, by performing surface water-soluble modification, 22 mg of the final product was obtained, with a sample yield of 41% and product radioactivity of 38.7 µCi, so that the labeling rate was 38.7/(218 × 0.41) = 43.3%. The labeling rate was further confirmed to be 44% using the calibrator. Considering that Na₇Zr₆F₃₁:Yb/Er/⁸⁹Zr would be gradually hydrolyzed when it was exposed to water, the decrease in the labeling rate indicated that the sample had the ability to be hydrolyzed.

The schematic diagram and the function of the prepared nanomaterial are shown in FIG. 16. The rare-earth doped inorganic nano-fluorescent PET imaging agent of the present disclosure is composed of an inner core with medical isotope radioactivity and a functional shell layer on the surface of the inner core. The material is characterized in that the radioactive inner core is simultaneously doped with rare-earth ions and radioactive isotopes, wherein the rare-earth ions can provide fluorescent imaging capability of a full spectrum from ultraviolet to visible to near-infrared light under a specific excitation light source (such as excitation at 980 nm). The medical radionuclide can provide effects of PET imaging and radiotherapy for the material. The functional shell layer is wrapped outside the material, so that the requirements of the material for different biomedical applications can be met. For example, a water-soluble ligand on the surface of the material can enhance the dispersibility of the material in the blood, which can meet the imaging requirements of blood diseases such as thrombus; the modification of the surface of the material with a tumor-targeting ligand can achieve the targeted radiotherapy and imaging at a tumor position. In addition, the imaging agent can be decomposed and removed from organisms after the corresponding task is completed, which effectively reduces the amount of accumulation in organisms, reduces biotoxicity, and meets the requirements of biosafety.

### Experimental Example 1: Detection of Hydrolysis Performance of Rare-Earth Fluorescent PET Nano-Imaging Agent

### (1) Detection of hydrolysis performance of Na₇Zr₆F₃₁:Yb/Er/⁸⁹Zr

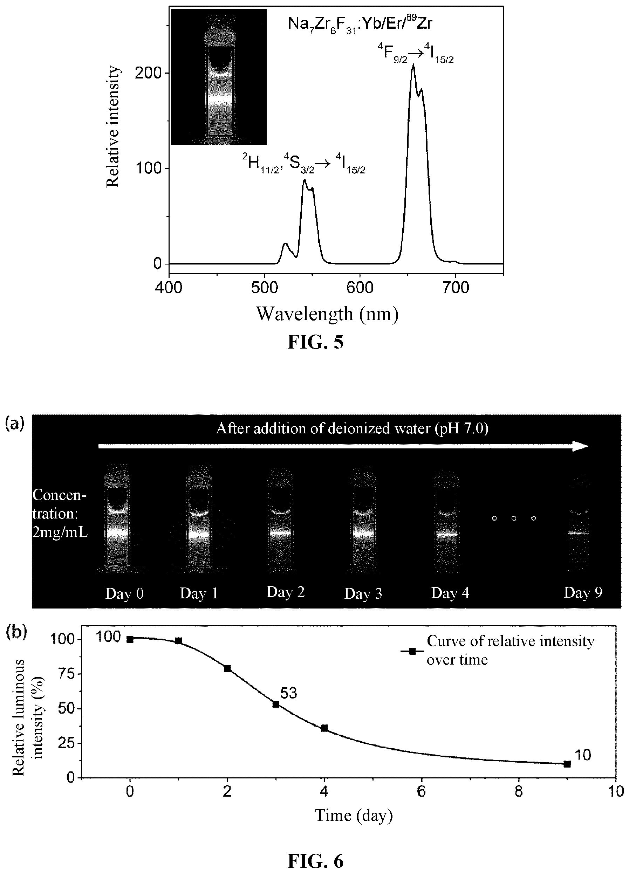

The Na₇Zr₆F₃₁:Yb/Er/⁸⁹Zr-based fluorescent PET nano-imaging agent prepared in Example 1 was formulated into a 2 mg/mL aqueous solution, and the solution exhibited a yellow-green luminous pattern under the excitation of 980 nm laser. As shown in FIG. 5, the sample had a characteristic up-conversion emission spectrum of Er³⁺, wherein emission peaks corresponded to green light emission caused by ²H_{11/2},⁴S_{3/2} → ⁴I_{15/2} transition and red light emission caused by 4F_{9/2} → ⁴I_{15/2} transition, respectively. The luminous intensity was gradually decreased over time. The luminous intensity of the solution was measured and luminescence photographs (shown in FIG. 6(a)) were recorded in time intervals. The half-life for the decay of the luminous intensity was exactly 3 days (shown in FIG. 6(b), the luminous intensity decayed to 53% of the initial), which was matched with the half-life of ⁸⁹Zr, and by day 9, the luminous intensity decayed to 10% of the initial. As can be seen, the Na₇Zr₆F₃₁:Yb/Er/⁸⁹Zr-based fluorescent PET nano-imaging agent of Example 1 has the characteristic of slow hydrolysis matched with the half-life of ⁸⁹Zr.

### (2) Detection of hydrolysis performance of K₃ZrF₇:Yb/Er/⁸⁹Zr

Considering that the K₃ZrF₇:Yb/Er/⁸⁹Zr-based nanomaterial of Example 2 was hydrolyzed much faster than the Na₇Zr₆F₃₁:Y⁻b/Er/⁸⁹Zr-based nanomaterial described in Example 1, the nanomaterial prepared in Example 2 was dried to a solid powder and tested for hydrolysis performance.

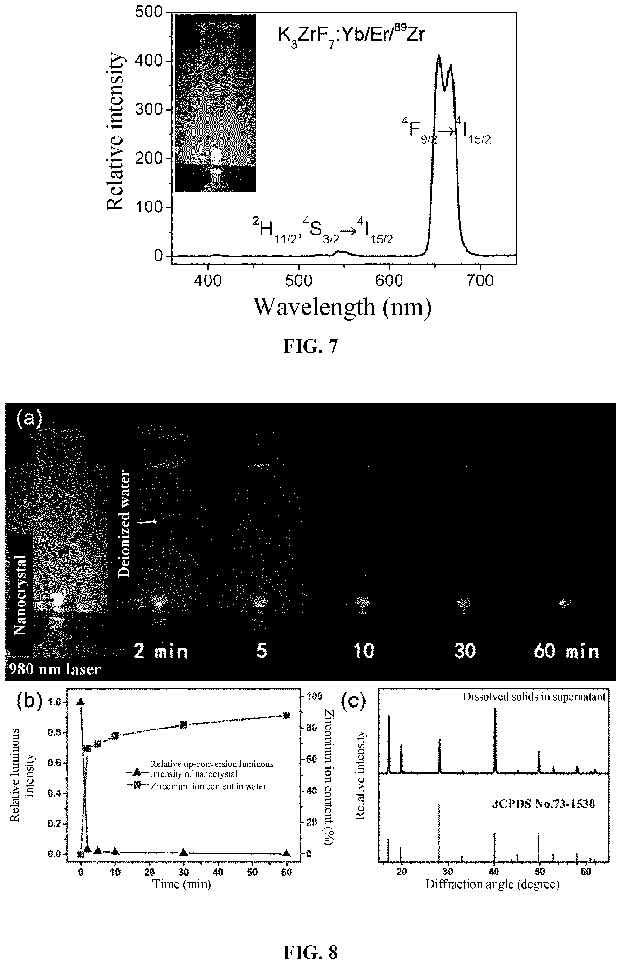

The K₃ZrF₇:Yb/Er/⁸⁹Zr-based nanomaterial prepared in Example 2 was dried into a powder, and under the excitation of 980 nm laser, the solid powder exhibited strong up-conversion red light emission (as shown in FIG. 7), namely corresponding to ⁴F_{9/2} → ⁴I_{15/2} electric dipole transition of Er³⁺, while green light emission was much weaker because ²H_{11/2},⁴S_{3/2} → ⁴I_{15/2} magnetic dipole transition of Er³⁺ was affected by transition forbiddenness caused by high symmetry of a cubic phase. The solid powder was immersed in a certain amount of deionized water. The red light emission of the solid powder gradually diminished over time, and no light emission was observed in the supernatant. As shown in FIG. 8(a), the luminous intensity of the solid and the content of ions in the supernatant were measured, and luminescence photographs were recorded, in time intervals. When the powder was immersed in deionized water, the relative luminous intensity was rapidly reduced within 1 h until substantially no light emission was observed, and ions in the lower layer were almost completely dissolved in deionized water in the upper layer, as shown in FIG. 8(b). FIG. 8(c) is a powder diffraction pattern of the solid after evaporation of the supernatant after 2 days of immersion, in which the positions of diffraction peak of the solid were still consistent with those in the PDF standard card of K₃ZrF₇ (JCPDS No. 73-1530). This suggested that the nanomaterial prepared in Example 2 was decomposed well in deionized water. As can be seen, the K₃ZrF₇:Yb/Er/⁸⁹Zr-based fluorescent PET nano-imaging agent of Example 2 has the characteristic of rapid hydrolysis.

### Experimental Example 2: Biofluorescent Imaging Effect of K₃ZrF₇:Yb/Er/⁸⁹Zr-Based Rare-Earth Fluorescent PET Nano-Imaging Agent

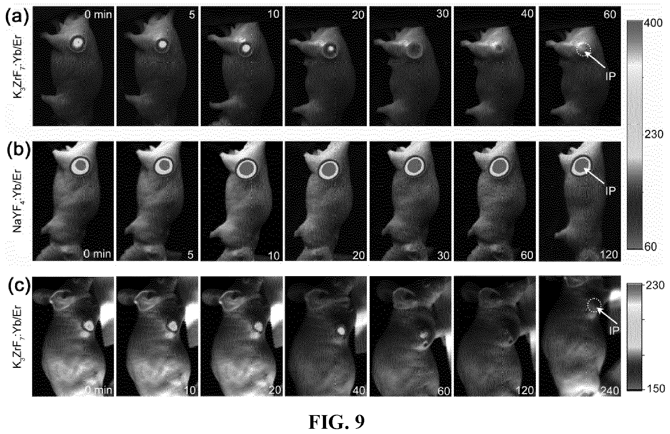

A trace amount of peanut oil solution (50 mg/mL) of K₃ZrF₇:Yb/Er/⁸⁹Zr-based nanomaterial of Example 2 was subcutaneously injected into a nude mouse (supplied by Beijing Vital River Laboratory Animal Technology Co., Ltd.), and imaging was performed under the excitation of excitation light at 980 nm using a CCD imaging system. As shown in FIG. 9(a), fluorescence could be observed at a location where the nano-imaging agent was injected, and it gradually diminished over time and was substantially no longer observed at about 40 min.

A trace amount of peanut oil solution (50 mg/mL) of NaYF₄:Yb/Er-based nanomaterial of Comparative Example 1 was subcutaneously injected into a nude mouse, and imaging was performed under the excitation of excitation light at 980 nm using a CCD imaging system. As shown in FIG. 9(b), fluorescence could be observed at a location where the nano-imaging agent was injected, with no significant changes over time, and it could still be clearly observed after 2 h.

A trace amount of peanut oil solution (50 mg/mL) of the K₃ZrF₇:Yb/Er/⁸⁹Zr-based nanomaterial of Example 2 was subcutaneously injected into a position of a 4T1 tumor tissue (in a weakly acidic environment) of a nude mouse, and imaging was performed under the excitation of excitation light at 980 nm using a CCD imaging system. As shown in FIG. 9(c), fluorescence could be observed at a location where the nano-imaging agent was injected, and it diminished over time and was substantially no longer observed at about 4 h.

This suggested that luminescence of the K₃ZrF₇:Yb/Er/⁸⁹Zr-based nano-imaging agent in organisms diminished over time, but can be maintained for a longer period of time in a weakly acidic tumor environment.

### Experimental Example 3: Imaging Effect of Na₇Zr₆F₃₁:Yb/Er/⁸⁹Zr-Based Rare-Earth Fluorescent PET Nano-Imaging Agent PET

The fluorescent PET nano-imaging agent prepared in Example 1 was injected into quarantine-compliant ICR mice (supplied by Beijing Vital River Laboratory Animal Technology Co., Ltd.) weighing about 20 g through the tail vein. Different doses of administration result in different imaging effects:
(1) The concentration of administration was 150 mg/kg, the radioactive dose was 4.26 mCi/kg, and PET imaging was then performed at time intervals of 1 h, 3 h, 6 h, 72 h, and 120 h.

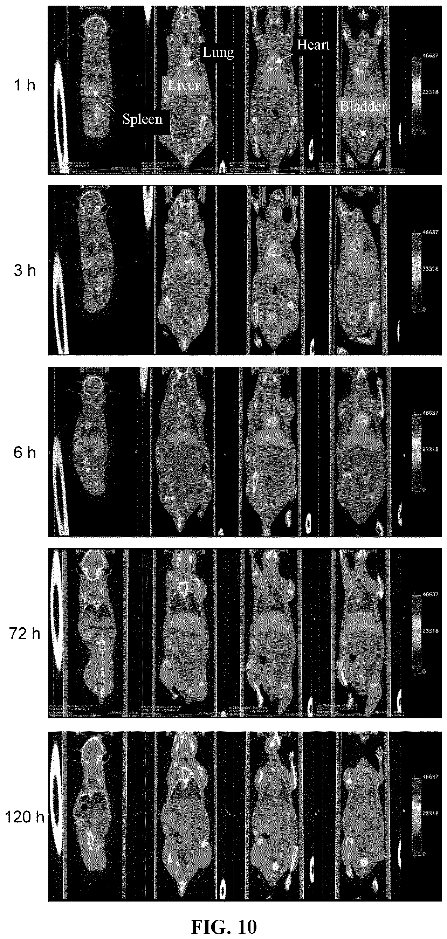

As shown in FIG. 10, the resulting PET images have good quality, and the distribution of the drug (referring to the PET nano-imaging agent) in the mouse could be clearly observed. Within 1 h after the start of the injection, the drug rapidly diffused throughout the body of the mouse to most organs including the heart, liver, spleen, lungs, and intestinal tract; a large amount of the drug was directly excreted to the outside of the body through the bladder and rectum within 3 h; in the first 3 days, the remaining drug gradually accumulated at positions of metabolic organs of the liver and spleen and was eliminated from the body in a mode of self-degradation of the drug into an ionic state and blood circulation, during which the slow hydrolysis characteristic of the drug could still provide clear PET imaging quality; at day 5, most of the drug had been eliminated from the body by degradation, and the remaining drug was insufficient to provide a clear PET image. This suggested that the fluorescent PET nano-imaging agent prepared in Example 1 could be degraded, metabolized, and eliminated from the body after the imaging task was completed.

(2) The concentration of administration was 75 mg/kg, the radioactive dose was 2.13 mCi/kg, and PET imaging was then performed at time intervals of 1 h, 3 h, and 6 h.

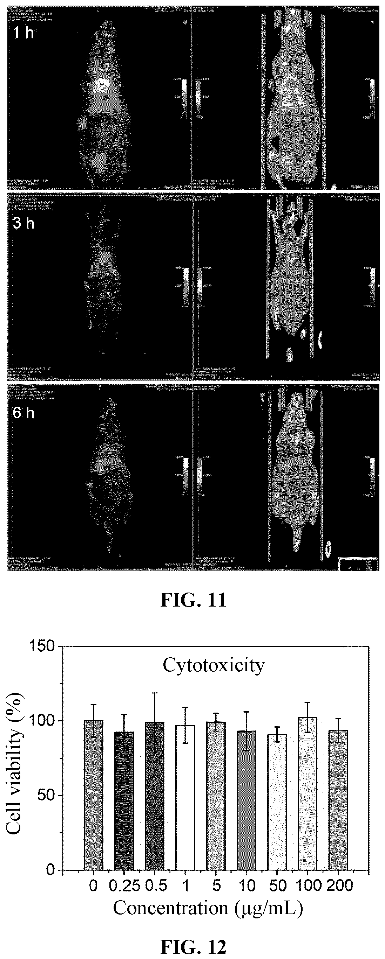

As shown in FIG. 11, the resulting PET images have good quality, and the distribution of the drug (referring to the PET nano-imaging agent) in the mouse could be clearly observed. Within 1 h after the start of the injection, the drug rapidly diffused throughout the body of the mouse to most organs including the heart, liver, spleen, lungs, and intestinal tract; most of the drug was metabolized through the bladder and rectum within 3 h; after 6 h, a small amount of the remaining drug finally accumulated at positions of metabolic organs such as the liver, spleen, and intestinal tract and are gradually eliminated from the body. The results showed that the fluorescent PET nano-imaging agent prepared in Example 1 could be eliminated from the body in a biological self-degradation mode within a short time at this dose, thereby reducing the radiation damage caused by nuclides to the body.

The experimental results showed that the fluorescent PET nano-imaging agent prepared in Example 1 could adjust the metabolic rate of the drug through the dose of administration, so that the appropriate dose of administration could be selected according to the actual application.

### Experimental Example 4: Biosafety of Na₇Zr₆F₃₁:Yb/Er/⁸⁹Zr-Based Rare-Earth Fluorescent PET Nano-Imaging Agent

### (1) Cytotoxicity test: (by an MTT colorimetric method and using human embryonic kidney cells 293T)

As shown in FIG. 12, the Na₇Zr₆F₃₁:Yb/Er/⁸⁹Zr-based nano-imaging agent prepared in Example 1 could maintain normal cell activity of human embryonic kidney cells 293T (from Beijing Dingguo Changsheng Biotech Co. Ltd., seeded with a cell suspension at a concentration of 50,000 cells/mL) in the concentration range of 0-0.2 mg/mL of administration, which indicated that the nano-imaging agent had very low cytotoxicity.

### (2) Acute Toxicity Test in Mice

In this test, the toxic response of ICR mice (provided by Beijing Vital River Laboratory Animal Technology Co., Ltd.) within 15 days was investigated by a maximum feasible dose method, through single intraperitoneal administration at doses of 0 mg/kg (negative control group), 12.5 mg/kg, 25.0 mg/kg, 50.0 mg/kg, 100.0 mg/kg, and 200.0 mg/kg and at a volume of 20 mL/kg, with normal saline as the solvent. The test subjects were 60 ICR mice, and grouping was performed according to the standard operating protocol for clinical trials (SOPO 309A), in which mice were divided into 6 groups of 10 mice each (half male (F) and half female (M)) according to the weights of the animals by randomized grouping.

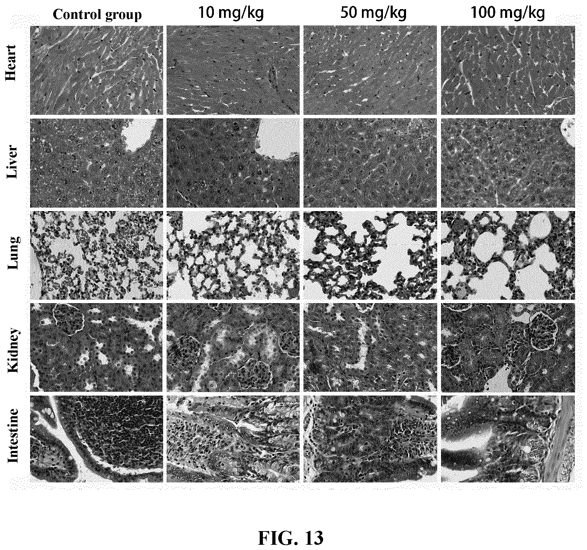

As shown in Tables 1 and 2, when the Na₇Zr₆F₃₁:Yb/Er/⁸⁹Zr-based nano-imaging agent prepared in Example 1 was administered at a concentration range of 0-200 mg/kg, the male/female mice showed no significant difference in body weight change from the control group and developed normally until maturation, and no abnormal responses were observed in the appearance, nervous system, respiratory and circulatory systems, genitourinary system, behavior and activity, and food and water intake of the male and female mice during the observation period (from day 1 to day 15). As shown in FIG. 13, no macroscopic lesions were observed in the dissection, and no abnormal lesions were observed in the heart, liver, lung, kidney and intestine tissues of all animals by histopathological examination.

**Table 1. Body weight statistics (g, x̅ ± s, n = 5) of female mice after single intraperitoneal injection of Na₇Zr₆F₃₁:Yb/Er/⁸⁹Zr in a toxicity test**

| Group | D1 | D15 |
|---|---|---|
| Negative control group | 19.2±2.10 | 26.8±1.81 |
| 12.5 mg/kg group | 19.0±2.10 | 26.1±1.07 |
| 25.0 mg/kg group | 19.2±1.97 | 26.5±1.95 |
| 50.0 mg/kg group | 19.2±1.69 | 26.3±1.82 |
| 100.0 mg/kg group | 18.9±1.79 | 27.2±2.39 |
| 200.0 mg/kg group | 19.4±2.17 | 26.4±2.75 |

| | | |
|---|---|---|
| *: compared with the negative control group, P < 0.05. | | |

**Table 2. Body weight statistics (g, x̅ ± s, n = 5) of male mice after single intraperitoneal injection of Na₇Zr₆F₃₁:Yb/Er/⁸⁹Zr in a toxicity test**

| Group | D1 | D15 |
|---|---|---|
| Negative control group | 24.2±1.63 | 33.1±2.48 |
| 12.5 mg/kg group | 23.8±1.99 | 33.7±4.03 |
| 25.0 mg/kg group | 23.9±1.24 | 32.5±2.59 |
| 50.0 mg/kg group | 23.7±1.65 | 33.5±3.75 |
| 100.0 mg/kg group | 24.0±1.17 | 33.2±4.34 |
| 200.0 mg/kg group | 23.8±1.29 | 32.6±1.75 |

| | | |
|---|---|---|
| *: compared with the negative control group, P < 0.05. | | |

### (3) Subacute Toxicity Test in Rats

In this test, the toxic response of SD rats (provided by Beijing Vital River Laboratory Animal Technology Co., Ltd.) was investigated by a maximum feasible dose method, through administration through the tail vein every three days for 15 consecutive days, at doses of 0 mg/kg (negative control group), 2 mg/kg, 5 mg/kg, and 10 mg/kg and at a volume of 20 mL/kg, with normal saline as the solvent. The test subjects were 40 SD rats, and grouping was performed according to the standard operating protocol for clinical trials (SOPO 309A), in which rats were divided into 4 groups of 10 rats each (half male (F) and half female (M)) according to the weights of the animals by randomized grouping.

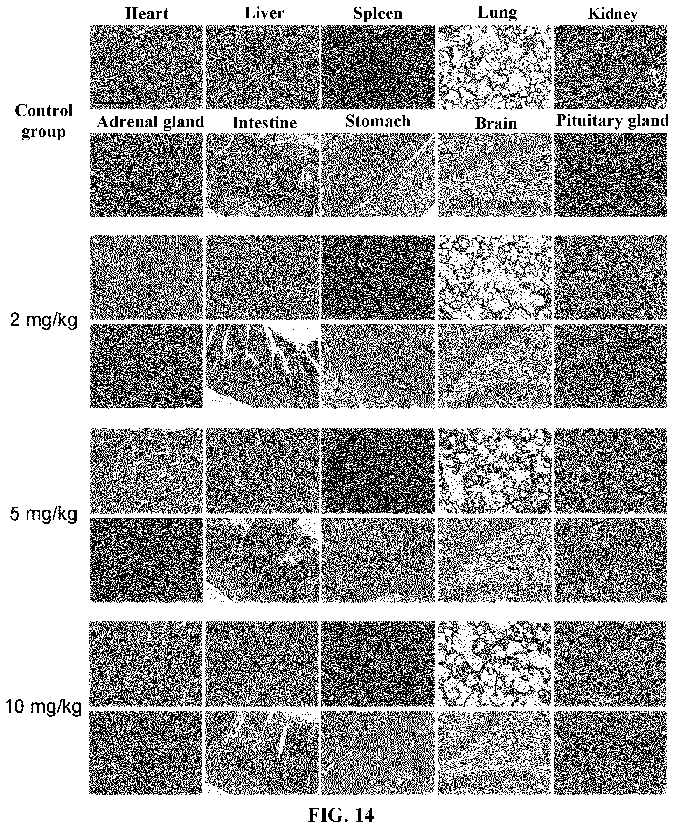

As shown in Tables 3 and 4, when the Na₇Zr₆F₃₁:Yb/Er/⁸⁹Zr-basednano-imaging agent prepared in Example 1 was administered at a concentration range of 0-10 mg/kg, the male and female rats showed no significant difference in body weight change from the control group and developed normally until maturation, and no abnormal responses were observed in the appearance, nervous system, respiratory and circulatory systems, genitourinary system, behavior and activity, and food and water intake of the male and female rats during the observation period (from day 1 to day 15). As shown in FIG. 14, no macroscopic lesions were observed in the dissection, and no abnormal lesions were observed in the heart, liver, lung, kidney and intestine tissues of all animals by histopathological examination.

**Table 3. Body weight statistics (g, x̅ ± s, n = 5) of female SD rats after 2-week intravenous injection of Na₇Zr₆F₃₁:Yb/Er/⁸⁹Zr in a subacute toxicity test**

| Group | D1 | D15 |
|---|---|---|
| Negative control group | 190.4±11.25 | 229.4±16.17 |
| 2.0 mg/kg group | 191.7±8.76 | 222.8±12.76 |
| 5.0 mg/kg group | 196.0±16.36 | 231.0±16.09 |
| 10.0 mg/kg group | 193.5±8.20 | 226.4±10.21 |

| | | |
|---|---|---|
| *: compared with the negative control group, P < 0.05. | | |

**Table 4. Body weight statistics (g, x̅ ± s, n = 5) of male SD rats after 2-week intravenous injection of Na₇Zr₆F₃₁:Yb/Er/⁸⁹Zr in a subacute toxicity test**

| Group | D1 | D15 |
|---|---|---|
| Negative control group | 214.2±9.14 | 315.4±20.80 |
| 2.0 mg/kg group | 216.4±13.63 | 330.4±22.18 |
| 5.0 mg/kg group | 214.3±8.78 | 307.0±19.35 |
| 10.0 mg/kg group | 213.8±7.47 | 308.4±15.11 |

| | | |
|---|---|---|
| *: compared with the negative control group, P < 0.05. | | |

### (4) Allergy Test in Guinea Pigs

In this test, the allergic response of guinea pigs (provided by Beijing Vital River Laboratory Animal Technology Co., Ltd.) was investigated by intraperitoneal injection for sensitization for three times, and finally stimulation by foot intravenous injection at doses of 0 mg/kg (negative control group), 0.5 mg/kg, 1 mg/kg, and 2 mg/kg and at a volume of 20 mL/kg, with an ovalbumin injection group as a positive control group, and with normal saline as the solvent. The test subjects were 80 guinea pigs, and grouping was performed according to the standard operating protocol for clinical trials (SOPO 309A), in which the guinea pigs were divided into 5 groups of 8 guinea pigs each (half male (F) and half female (M)) according to the weights of the animals by randomized grouping.

As shown in FIG. 15, when the Na₇Zr₆F₃₁:Yb/Er/⁸⁹Zr-basednano-imaging agent prepared in Example 1 was administered at a concentration range of 0-2 mg/kg, the male and female guinea pigs showed no significant difference in body weight change from the control group and developed normally until maturation, and no abnormal responses were observed in the appearance, nervous system, respiratory and circulatory systems, genitourinary system, behavior and activity, and food and water intake of the male and female guinea pigs during the observation period (from day 1 to day 15). As shown in Tables 5 and 6, no significant positive allergic response was observed in comparison with the positive allergic response standard.

**Table 5. Allergic response score statistics (n=8) of female guinea pigs after administration of Na₇Zr₆F₃₁:Yb/Er/⁸⁹Zr**

| Intensity of allergic response | Negative control group | 0.5 mg/kg group | 1.0 mg/kg group | 2.0 mg/kg group | Positive control group |
|---|---|---|---|---|---|
| Grade 0, negative | 0 | 0 | 0 | 0 | 0 |
| Grade 1-4, weak positive | 0 | 0 | 0 | 0 | 0 |
| Grade 5-10, positive | 0 | 0 | 0 | 0 | 0 |
| Grade 11-19, strong positive | 0 | 0 | 0 | 0 | 0 |
| Grade 20, extremely strong positive | 0 | 0 | 0 | 0 | 8 (4/4) |

**Table 6. Allergic response score statistics (n=8) of male guinea pigs after administration of Na₇Zr₆F₃₁:Yb/Er/⁸⁹Zr**

| Intensity of allergic response | Negative control group | 0.5 mg/kg group | 1.0 mg/kg group | 2.0 mg/kg group | Positive control group |
|---|---|---|---|---|---|
| Grade 0, negative | 0 | 0 | 0 | 0 | 0 |
| Grade 1-4, weak positive | 0 | 0 | 0 | 0 | 0 |
| Grade 5-10, positive | 0 | 0 | 0 | 0 | 0 |
| Grade 11-19, strong positive | 0 | 0 | 0 | 0 | 0 |
| Grade 20, extremely strong positive | 0 | 0 | 0 | 0 | 8 (4/4) |

Those skilled in the art can expect that when Ti is used to replace Zr and Hf, Li, Rb or Cs is used to replace Na or K, other stable isotopes of rare-earth elements (such as Y, Sc, Yb, Er, Tm, Ho, Gd, Eu, Tb, Sm, Dy, Ce, Nd, La, Pr or Lu) are used to replace Yb and Er, and other radioactive medical isotopes (such as ⁹⁰Y, ¹⁷⁷Lu, ¹⁵³Sm, ²²³Ra and ¹¹¹In) are used to replace ⁸⁹Zr, the resulting rare-earth doped fluorescent nanomaterials have the same or similar properties as those in the examples, and can also be used as PET imaging agents.

The examples of the present disclosure have been described above. However, the present disclosure is not limited to the above examples. Any modification, equivalent, improvement and the like made without departing from the spirit and principle of the present disclosure shall fall within the protection scope of the present disclosure.

## Claims

1. A nanomaterial comprising an inner core, wherein the inner core has a structural general formula of MₓT_{y}F_{x+4y}:Ln/R;
wherein M is an alkali metal element selected from one or more of Li, Na, K, Rb, and Cs;
T is a transition metal element selected from one or more of Ti, Zr, and Hf;
1 ≤ x ≤ 7, 1 ≤ y ≤ 6;
Ln is a stable isotope of rare-earth elements selected from one or more of Y, Sc, Yb, Er, Tm, Ho, Gd, Eu, Tb, Sm, Dy, Ce, Nd, La, Pr, and Lu;
R is a radioactive medical isotope selected from one or more of ⁹⁰Y, ¹⁷⁷Lu, ¹⁵³Sm, ²²³Ra, ¹¹¹In, and ⁸⁹Zr.

2. The nanomaterial as claimed in claim 1, wherein R is distributed at least inside a crystal of the inner core;
preferably, R is distributed in the inner core at an abundance of at least 5 mCi/g;
preferably, the inner core is selected from materials having the following structural formula: K₃ZrF₇:Yb/Er/⁸⁹Zr, K₂ZrF₆:Yb/Er/⁸⁹Zr, KZrF₅:Yb/Er/⁸⁹Zr, K₃HfF₇:Yb/Er/⁸⁹Zr, K₂HfF₆:Yb/Er/⁸⁹Zr, K₃Zr_{0.5}Hf_{0.5}F₇:Yb/Er/⁸⁹Zr, Na₃ZrF₇:Yb/Er/⁸⁹Zr, Na₂ZrF₆:Yb/Er/⁸⁹Zr, Na₅Zr₂F₁₃:Yb/Er/⁸⁹Zr, Na₇Zr₆F₃₁:Yb/Er/⁸⁹Zr, Na₃HfF₇:Yb/Er/⁸⁹Zr, Na₅Hf₂F₁₃:Yb/Er/⁸⁹Zr, Na₃Zr_{0.5}Hf_{0.5}F₇:Yb/Er/⁸⁹Zr, Na₇Hf₆F₃₁:Yb/Er/⁸⁹Zr, Li₄ZrF₈:Yb/Er/⁸⁹Zr, Li₂ZrF₆:Yb/Er/⁸⁹Zr, Li₃Zr₄F₁₉:Yb/Er/⁸⁹Zr, Li₂HfF₆:Yb/Er/⁸⁹Zr, Rb₅Zr₄F₂₁:Yb/Er/⁸⁹Zr, Rb₂ZrF₆:Yb/Er/⁸⁹Zr, Cs₂ZrF₆:Yb/Er/⁸⁹Zr, RbHfF₅:Yb/Er/⁸⁹Zr, Rb₂HfF₆:Yb/Er/⁸⁹Zr, Cs₂HfF₆:Yb/Er/⁸⁹Zr, K₂TiF₆:Yb/Er/⁸⁹Zr, or Na₂TiF₆:Yb/Er/⁸⁹Zr; exemplarily K₃ZrF₇:Yb/Er/⁸⁹Zr, Na₇Zr₆F₃₁:Yb/Er/⁸⁹Zr, or Na₅Hf₂F₁₃:Yb/Er/⁸⁹Zr.

3. The nanomaterial as claimed in claim 1 or 2, wherein the inner core has a crystal structure and has a dimension of 5-100 nm.

4. The nanomaterial as claimed in any one of claims 1-4, further comprising a functional shell layer, wherein the functional shell layer is located on the surface of the inner core, for example, the surface of the inner core is partially or completely coated with the functional shell layer;
preferably, the functional shell layer is a functional shell layer composed of an organic material, a functional shell layer composed of an inorganic material, or an organic/inorganic hybrid functional shell layer;
preferably, the organic ligand is selected from a water-soluble functional ligand, an oil-soluble functional ligand, an amphiphilic functional ligand, or other functional ligands with pharmaceutical functions;
preferably, the functional shell layer has a thickness of 1-10 nm;
preferably, the nanomaterial has a dimension greater than that of the inner core.

5. A preparation method for the nanomaterial as claimed in any one of claims 1-4, wherein the preparation method is a coprecipitation method, a thermal decomposition method, a hydrothermal method, or a sol-gel method, e.g., a coprecipitation method, for preparing the nanomaterial;
preferably, the coprecipitation method for preparing the nanomaterial comprises the following steps:
(1) using a stable isotope T source, a non-radioactive rare-earth source, a radioactive isotope source, long-alkyl-chain organic acid, octadecene or trioctylamine, an alkali metal source, and a fluorine source as starting materials, and obtaining an inner core by a coprecipitation method; and
(2) optionally, modifying the inner core with a functional shell layer to obtain the nanomaterial.

6. The preparation method as claimed in claim 5, wherein a T element in the stable isotope T source is one or more of Ti, Zr, and Hf, and is exemplarily selected from Zr or/and Hf;
preferably, the stable isotope T source is selected from one or more of the following: zirconium acetylacetonate, hafnium acetylacetonate, zirconium acetate, titanium tetrachloride, zirconium chloride, and hafnium chloride;
preferably, when the non-radioactive rare-earth source is selected from a Yb ion stable isotope source and an Er ion stable isotope source, the Yb ion and the Er ion are in a molar ratio of (10-30) : (0.5-4);
preferably, the stable isotope T source, the non-radioactive rare-earth source, the long-alkyl-chain organic acid, and the octadecene are in a mole-to-volume ratio of (0.1-0.49) mmol : (0.01-0.4) mmol : (5-10) mL : (5-30) mL;
preferably, the stable isotope T source, the non-radioactive rare-earth source, the long-alkyl-chain organic acid, and the trioctylamine are in a mole-to-volume ratio of (0.1-0.49) mmol : (0.01-0.4) mmol : (5-10) mL : (5-30) mL;
preferably, the radioactive isotope source and the stable isotope T source are in a radioactivity-to-mole ratio of (1-10) mCi : (0.1-0.49) mmol.

7. The preparation method as claimed in claim 5 or 6, wherein the step (1) comprises:
(a) mixing the stable isotope T source, the non-radioactive rare-earth source, the radioactive isotope source, the long-alkyl-chain organic acid, and the octadecene, or mixing the stable isotope T source, the non-radioactive rare-earth source, the radioactive isotope source, the long-alkyl-chain organic acid, and the trioctylamine in a reaction container until dissolution to obtain a solution a; and
(b) adding the alkali metal source and the fluorine source to the solution a, and performing a coprecipitation reaction to obtain the inner core;
preferably, the coprecipitation reaction in the step (b) is performed at a temperature of 260-340 °C for 0.3-2 h.

8. The preparation method as claimed in claim 7, wherein the step (2) can be modifying the inner core with different functional shell layers according to the usage requirements,
preferably modifying the inner core with a functional shell layer composed of a water-soluble ligand;
preferably, the step of modifying the inner core comprises: dispersing the inner core in a mixed solvent, adding trimethyloxonium tetrafluoroborate for a reaction to remove oil-soluble substances on the surface of the inner core, precipitating the resulting solution, re-dispersing the solution in N,N-dimethylformamide (DMF), adding a functional ligand solution (preferably a water-soluble functional ligand solution) under stirring, and performing a reaction to obtain the nanomaterial.

9. Use of the nanomaterial as claimed in any one of claims 1-4 as a rare-earth fluorescent PET imaging agent,
preferably as a specific rare-earth fluorescent PET imaging agent and/or therapeutic agent for diseases including, but not limited to cancer, neurodegenerative diseases, and cardiovascular and cerebrovascular diseases.

10. A PET imaging agent comprising the nanomaterial as claimed in any one of claims 1-4.
